# EUROPEAN PATENT SPECIFICATION

(11) **EP 1 187 930 B1**
(45) Date of publication and mention of the grant of the patent: **02.08.2006**
(21) Application number: 00937871.2
(22) Date of filing: 26.05.2000
(51) Int. Cl.: C12P 1/00, C12P 13/04, C12P 13/22, C12N 1/20

(54) **A GLOBAL REGULATOR USED TO ENHANCE THE SYNTHESIS OF AROMATIC SUBSTANCES**
GLOBALER REGULATOR, VERWENDET ZUR ERHöHUNG DER SYNTHESE VON AROMATISCHEN SUBSTANZEN
REGULATEUR GLOBAL UTILE POUR RENFORCER LA SYNTHESE DE SUBSTANCES AROMATIQUES

(30) Priority: 28.05.1999 US 136441 P
(43) Date of publication of application: 20.03.2002
(73) Proprietor: THE UNIVERSITY OF NORTH TEXAS HEALTH SCIENCE CENTER AT FORT WORTH, Fort Worth TX 76107-2699 (US)
(72) Inventor: ROMEO, Tony, Arlington, TX 76017 (US); TATARKO, Matthew, Fort Worth, TX 76107 (US)
(74) Representative: Wright, Simon Mark
(86) International application number: PCT/US2000/014668
(87) International publication number: WO 2000/073484

(56) References cited:
- US-A- 4 681 852
- US-A- 5 168 056
- US-A- 5 684 144
- US-A- 5 776 736
- US-A- 5 906 925
- PATNAIK R. ET AL.: 'Engineering of Escherichia coli central metabolism for aromatic metabolite production with near theoretical yield' APPL. ENVIRON. MICRO., vol. 60, no. 11, November 1994, pages 3903 - 3908, XP002931420
- PATNAIK R. ET AL.: 'Pathway engineering for production of aromatics in Escherichia coli: Confirmation of Stoichiometric analysis by independent modulation of AroG, TktA and Pps activities' BIOTECHNOLOGY AND BIOENGINEERING, vol. 46, no. 4, May 1995, pages 361 - 370, XP002931419

## Description

### STATEMENTS OF RIGHTS TO INVENTIONS MADE UNDER FEDERALLY SPONSORED RESEARCH

This invention was supported by a grant from the National Science Foundation (MCB 9726197), the United States government may have certain rights to the invention.

### TECHNICAL FIELD

The present invention relates to a microorganism which is genetically engineered to increase carbon flow into the production of precursors and metabolic intermediates of the common aromatic pathway and pathways branching therefrom as well as to methods of increasing such carbon flow.

### BACKGROUND ART

Approaches for the metabolic engineering of microorganisms have involved elimination of enzymes leading to by-products, deregulation of allosteric enzymes, derepression or overexpression of specific genes involved in the pathway, and the introduction of genes from other organisms using recombinant DNA and other approaches. Bailey (1991) Science 252:1668-1675. In part, these measures are required to overcome endogenous regulatory mechanisms, which function to match the flux through specific metabolic pathways with the immediate needs of the organism. Free-living bacteria likewise regulate metabolism at specific steps of individual pathways, but also coordinate flux through sets of pathways by global regulatory networks. Global regulatory systems modulate the expression of numerous genes located throughout the bacterial genome, permitting the overall physiological, metabolic, and developmental status of the organism to respond rapidly and sometimes dramatically to changes in the environment. Neidhardt and Savageau (1996) Regulation beyond the operon, In, *Escherichia coli* and *Salmonella:* Cellular and Molecular Biology, Vol. 2 2^{nd} edn. Neidhardt et al. Eds. Am. Soc. Microbiol. Washington, D.C. pp. 1310-1324. The potential of these powerful regulatory systems is yet to be realized for microbial strain construction.

The biological production of aromatic amino acids and related compounds has clear advantages over chemical synthesis, e.g. the biological processes are more environmentally sound and utilize renewable resources. Berry (1996) Trends Biotechnol. 14:250-256. However, the microbial processes are limited by the modest yield at which substrate is converted to end product. Bacteria use the shikimate or common aromatic pathway for the synthesis of aromatic amino acids, folates and other aromatic metabolites. Draths et al. (1992) J. Am. Chem. Soc. 114:3956-3962. This pathway starts with the condensation of the central carbon intermediates phosphoenolpyruvate (PEP) and erythrose-4-phosphate (E4P) to form 3-deoxy-D-arabino-heptulosonate-7-phosphate (DAHP), in a reaction that is catalyzed by three isozymes of DAHP synthase, which are feedback inhibited by either phenylalanine (AroG), tyrosine (AroF) or tryptophan (AroH). A second PEP molecule is incorporated at a later step to form the intermediate 5-enolpyruvoylshikimate-3-phosphate (EPSP). Not until EPSP is converted to chorismate does this central pathway branch to permit the biosynthesis of phenylalanine, tyrosine, and tryptophan. In phenylalanine synthesis, the formation of prephenate from chorismate is catalyzed by chorismate mutase (PheA), which also is feedback inhibited by phenylalanine.

The aromatic pathway of *Escherichia coli* been the subject of prior genetic engineering to increase productivity and yield, and to expand the range of syntheses to non-native compounds such as indigo, indoleacetic acid, catechols and quinoid derivatives. Berry (1996); and Flores et al. (1996) Nature Biotechnol. 14:620-623. The regulatory enzymes of the shikimate pathway have been overproduced and their feedback mechanisms have been deregulated. U.S. Patent No. 4,681,652. Enzymes which generate the central carbon precursors of aromatic compounds, PEP and E4P, have been individually overproduced, i.e. PEP synthetase (Pps) and transketolase (TktA). Patnaik and Liao (1994) Appl. Environ. Microbiol. 60:3903-3908; US Patent No. 5,906,925; and Draths et al. (1992) J. Am. Chem. Soc. 114: 3956-3962, respectively. Systems which deplete PEP have been disrupted, including the two pyruvate kinases (PykF and PykA) (Gosset et al. (1996) J. Ind. Microbiol. 17:47-52), which convert PEP to pyruvate, and the PEP:glucose phosphotransferase system (PTS) (Flores et al. (1996)), which expends one PEP per glucose transported. Because the pathways of central carbon metabolism are interconnected numerous additional metabolic reactions may affect these precursors.

Over the past several years, the inventors have elucidated a global regulatory system that controls flux through the central carbon pathways, as well as many other physiological properties of *E. coli*, Csr or carbon storage regulator. Romeo (1998) Mol. Microbiol. 29:1321-1330; and EP application No. 98733282.0. The effector of this regulatory system is a small protein known as CsrA, whose novel mechanism for global regulation involves binding to specific mRNAs, rather than to specific DNA sequences. Liu et al. (1995) J. Bacteriol. 177:2663-2672; Liu et al. (1997) J. Biol. Chem. 272:17502-17510; and Liu and Romeo (1997) J. Bacteriol 179:4639-4642. Disruption of the *csrA* gene enhances the levels of gluconeogenic and glycogen biosynthetic enzymes, decreases glycolytic enzymes, and consequently perturbs the cellular concentrations of central carbon intermediates. Sabnis et al. (1995) J. Biol. Chem. 270:29096- 29104. CsrA modulates three enzymes that participate directly in PEP metabolism; PykF is positively-regulated, while PEP carboxykinase (PckA) and PEP synthetase (Pps), which generate PEP from oxaloacetate and pyruvate, respectively, are negatively regulated. Several enzymes that indirectly affect PEP are also regulated by CsrA. Thus, a *csrA* mutation causes an increase in PEP. Notably, *csrA* exhibits little or no effect on two genes of the pentose phosphate pathway, which generates E4P. Sabnis et al. (1995) J. Biol. Chem. 270:29096-29104.

### DISCLOSURE OF THE INVENTION

It has now been found that genetic elements which provide for disruption of the *csrA* gene can be advantageously employed in combination with genetic elements which cause increased E4P production to significantly increase production of compounds of the aromatic pathway including native and non-native compounds stemming from this pathway and particularly compounds that rely on chorismate as a precursor. In a particular application of this method, it has been found that disruption of csrA produces sufficient PEP to convert an increased amount of E4P resulting from over expression of the transketolase gene to DAHP (in a first condensation reaction) as well as sufficient PEP to produce EPSP from shikimate-3-phosphate (in a second condensation reaction) in strains engineered to optimize the aromatic pathway, including carbon flow through shikimate 3-phosphate and EPSP to chorismate.

Accordingly, disruption of the csrA gene in combination with over-expression of transketolase can advantageously be employed, in conjunction with other mutations which optimize the shikimate pathway to optimize carbon flow through chorismate (or one of the precursors of chorismate) where PEP is otherwise available in limiting quantities, for example, in strains optimized for phenylalanine production, or production of other aromatic amino acids. For example, in strains optimized for production of phenylalanine, the combined effect of a csrA description and E4P over-expression results in a more than 2 fold increase in phenylalanine production.

According to one aspect, the invention is directed to a microorganism which is genetically adapted to produce, under suitable culture conditions, particularly suitable nutrient supply, enhanced amounts of compounds flowing through the common aromatic pathway through at least shikimate-3-phosphate and 5-enolpyruvoylshikimate-3-phosphate (EPSP), said micro-organism comprising a first genetic element which decreases the amount of functional CsrA protein and a second genetic element which increases the amount of D-erythrose-4-phosphate. The micro-organism is thereby adapted to produce sufficient amounts of PEP to convert an increased amount of E4P to DAHP (in a first condensation reaction) and to convert resulting shikimate-3-phospate to EPSP (in a second condensation reaction). Furthermore this micro-organism, in virtue of this combination of genetic elements, is adapted to produce sufficient amounts of PEP for both condensation reactions in a micro-organism that is engineered to optimize the shikimate pathway. For example, a micro-organism in which DAHP synthase is feedback derepressed by one or more aromatic amino-acids, and in which, for example, increased amounts DAHP synthase and shikimate kinase are produced to optimize flow through the pathway to EPSP and preferably chorismate; or, for example, in a microorganism in which the pathway from chorismate to one of the aromatic amino acids, for example, phenylalanine, is also optimized, as more fully discussed below.

The term "functional" used with reference to a CsrA protein (gene product) refers to a protein which is capable of performing its normal function of decreasing the levels of gluconeogenic enzymes and glycogen biosynthesis enzymes etc., and in particular, capable of affecting enzymes and molecules which affect in turn the levels of production of PEP.

The term "increases" or "decreases" or similar terms used with references to enzymes refers to activity, levels, or amounts of molecules resulting from expression or introduction of genetic elements etc. is unless otherwise stated or implied, a relative term which is used by way of comparison to normal wild type levels for the wild-type microorganism or a specific reference microorganism which has been modified according to the invention. Unless otherwise stated or implied, such increase or decrease could be affected, directly or indirectly, within the range of activity, production or control mechanisms for the molecule in question.

The term "derepresses" and related forms of this term, when used with reference to a feedback inhibition mechanism is used to refer to a decrease in or prevention of such feedback inhibition.

In another aspect, the invention is directed to a microorganism that is genetically adapted to produce enhanced amounts of compounds flowing through the common aromatic pathway to a target compound which is intermediate in the pathway between DAHP and EPSP said microorganism comprising a first genetic element which decreases the available amount of functional CsrA protein and a second genetic element which increases the expression of E4P (relative to the wild-type microorganism), whereby said microorganism is adapted to channel excess PEP into the production of said target compound under conditions of suitably increased production of E4P, DAHP synthase and preferably other enzymes within the pathway leading to the target compound.

As used herein the term "genetic element" includes without limitation, a gene or part thereof (e.g. promoter) an extrachomosomal nucleic acid, for example, one or more plasmids etc., a nucleic acid incorporated into or interacting with the genome of the microorganism, for example a transposon insertion, or a mutation embodied in one or more altered or additional base pairs, or a deletion (for example, within or an existing genetic element, constituting in effect, a new genetic element). The term is not restricted to nucleic acids but may include a protein or other molecule, for example, a molecule introduced into the nutrient medium or derived from a co-cultured microorganism. The microorganism is preferably *E. coli*. For phenylalanine production the microorganism is preferably NST74 (ATCC #31884) as more fully discussed below.

The genetic element causing over expression of E4P is preferably the transketolase gene as disclosed in U.S. Patent No. 5,168,056. The genetic element responsible for decreasing the amount of csrA gene product is optionally the TR1-5 transposon insertion. However, as discussed below, it will be appreciated that any other methods of interfering with the expression or availability of functional CsrA protein to perform its native function may be contemplated for this purpose.

In another aspect, the invention is directed to a method of producing an increased amount of metabolic products of the shikimate pathway including EPSP and preferably chorismate by culturing, under suitable culture conditions, particularly suitable nutrient conditions, a micro-organism comprising a genetic element which decrease the amount of functional CsrA protein and a genetic element which causes an expression of E4P, whereby said microorganism is genetically adapted to produce sufficient amounts of PEP to convert E4P to DAHP and to convert resulting shikimate-3-phosphate to EPSP.

The importance and implementation of suitable culture conditions, if not explicit, is implicit throughout the disclosure and claims.

In yet another aspect, the invention is directed to a method of providing enhanced amounts of compounds flowing through the common aromatic pathway to a target compound which is intermediate in the pathway between DAHP and EPSP by culturing a microorganism under suitable nutrient conditions, said microorganism comprising a first genetic element which decreases the amount of functional CsrA protein and a second genetic element which increases the amount of E4P, whereby said microorganism is genetically adapted to channel excess PEP into the production of said target compound under conditions of suitably increased production or non-limiting amounts of E4P, DAHP synthase and preferably other enzymes within the pathway leading to the target compound. Preferably the enzyme responsible for catalyzing the synthesis of the next compound in the shikimate pathway, from the target compound, is blocked by a mutation or otherwise.

The term "culture" and related terms does not exclude any method known in the art for growing and/or maintaining microorganisms for the production of desired end-products.

### BRIEF DESCRIPTION OF THE DRAWINGS

Preferred embodiments of the invention will now be described with reference to the drawings, of which:
Figure 1 is a metabolic chart depicting the regulatory effects of *csrA* on intermediary carbon metabolism.
Figure 2 is a metabolic chart of the shikimate pathway.
Figure 3 is a metabolic chart depicting the production of phenylalanine and tyrosine from chorismate.
Figure 4 graphically depicts glucose uptake, and production of DHAP and phenylalanine in *E. coli* strains which overproduce aromatic enzymes. Strain identities are as follows: NST (●), NST[pAT1] (□), NST CSR (*csrA::kanR*) (■), and NST CSR[pAT1] (○). Cultures were grown in 100 ml modified MOPS medium containing 0.2% glucose as carbon source. Data points represent the averages of three determinations; error bars represent the standard deviations.
Figure 5 graphically depicts phenylalanine production (A) and percent yield by weight relative to glucose concentration (B). Strains were grown in 1ml modified MOPS medium containing the indicated concentrations of glucose. Strain identities were as follows: NST (■), NST[p AT1] 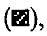 NST CSR (▩) and NST CSR[p AT1] 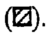

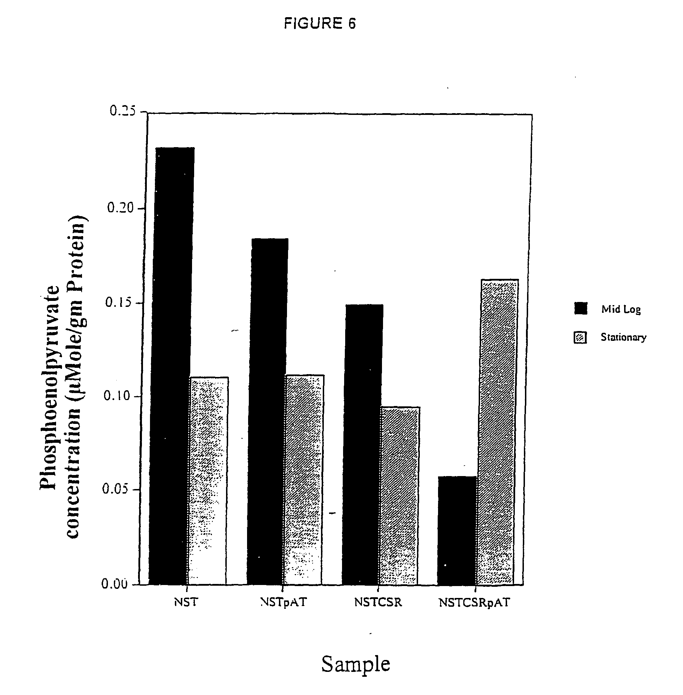

Figure 6 graphically depicts PEP levels in *E*. *coli* strains that overproduce aromatic pathway enzymes. Strains were grown to mid exponential phase (OD600 of 1.0; Mid log) or to early stationary phase in MOPS medium containing 0.2% glucose. PEP and protein levels were assayed as described in Example 1.

### BEST MODE FOR CARRYING OUT THE INVENTION

A few global regulatory factors mediate many of the extensive changes in gene expression that occur as *E. coli* enters the stationary phase. One of the metabolic pathways that is transcriptionally activated in the stationary phase is the pathway for glycogen biosynthesis. The csrA gene exerts pleiotropic effects by not only controlling glycogen biosynthesis but also controlling gluconeogenesis and exhibiting effects on cell size, surface (adherence) properties and motility. *E. coli* csrA thus encodes a global regulatory factor.

The nucleotide sequence of *E. coli csrA* is presented in Fig. 9 (SEQ ID NO:1) of U.S. Patent No. 5.684,144 (hereinafter the '144 patent). Fig. 9 (SEQ ID NO:1, SEQ ID NO:2 and SEQ ID NO:3) of the'144 patent depicts the nucleotide sequence of the *csrA* gene and the deduced amino acid sequence of the open reading frame (ORF) encoded therein. An arrow marks the site of the TR1-5 insertion mutation, which is between 421 and 422 bp, used to inactivate the csrA gene for the experiments described below and in Example 1. The TR1-5 insertion mutation is more fully described in Romeo et al. (1993) J. Bacteriol. 175:4744-4755. The insertion mutation is variously referred to herein as "TR1-5" "(*csrA*::kanR)" or "CSR" (when referring to a microorganism having the mutation). "NST" is used to refer to the NST74 strain of *E. coli* which is optimized for production of phenylalanine. NST74 is described in U.S. Patent No. 4,681,852.

As more fully described in the '144 patent, a plasmid clone of the native *csrA* gene capable of expressing the 61 amino acid *csrA* gene product strongly inhibits glycogen accumulation and affects the ability of cells to utilize certain carbon sources for growth. The regulated expression of the *csrA* gene and its gene product are useful for enhancing expression of products produced by alternative pathways. Such products include but are not limited to antibiotics, metabolites, organic acids, amino acids and a wide variety of industrially important compounds produced in bacterial fermentation systems.

### E. coli csrA gene and its Effects

Glycogen synthesis is one of a number of metabolic pathways that are induced in bacteria, for example, *E. coli* in the stationary phase. The accumulation of glycogen in the early stationary phase reflects at least two levels of regulation, allosteric regulation of the committed step of the biochemical pathway and enhanced expression of the structural genes for the pathway.

As more fully described in the '144 patent, the *csrA* gene encodes a trans-acting negative regulator of the expression of both *glgC* and *glgB*, essential enzymes of the glycogen pathway. The *csrA* mutation is pleiotropic, indicating that *csrA* encodes a global regulatory factor.

Glycogen synthesis is negatively controlled via the effects of the gene product of *csrA* (CsrA) on the expression of the operons *glgCAY*(P) and *glgBX*. The *csrA::kanR*. transposon insertion increased expression of *glgC* and *glgB*; however, it did not temporally alter the induction or change the shape of the induction curves for these genes. This indicates that CsrA functions in addition to the factors that mediate the growth phase response.

As described in more detail in the '144 patent, *csrA* is involved in the regulation of gluconeogenesis. The TR1-5 mutation in *csrA* caused overexpression of the gene pckA, as was evidenced by a twofold over-expression of a *pckA-lacZ* transcriptional fusion. The TR1-5 mutation, however, did not alter the shape of the *pckA-laZ* induction curve, a response that was similar to that of the glg genes.

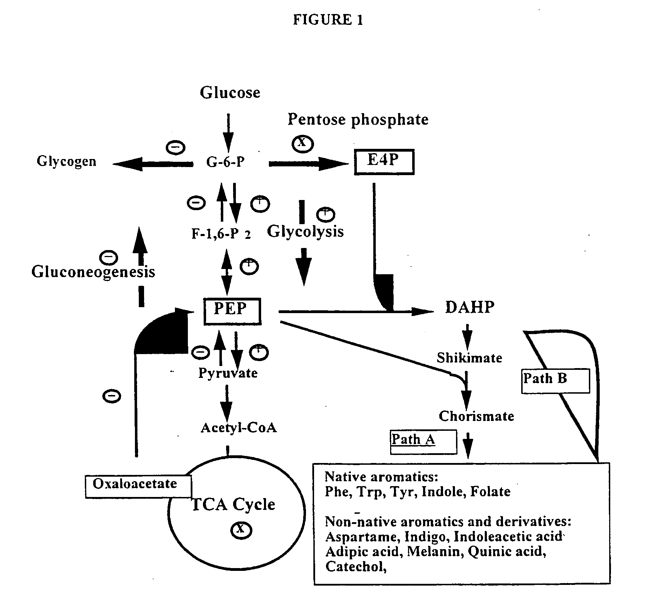

Referring to the Figures, Fig. 1 depicts the regulatory effects of *csrA* on intermediary carbon metabolism. For example, PEP is a precursor of glucose-6-phosphate via gluconeogenesis. Glucose-6-phosphate is, in turn, a precursor of glycogen. Gluconeogenesis and glycogen synthesis are also elevated in the *csrA* mutant and would compete with the shikimate pathway, which is involved in the synthesis of aromatic amino acids. As shown in Fig. 1, the *csrA* gene negatively regulates gluconeogenesis and glycogen synthesis and positively regulates glycolysis. Disruption of the *csrA* gene enhances the levels of gluconeogenic and glycogen biosynthetic enzymes, decreases glycolytic enzymes, and consequently perturbs the cellular concentrations of central carbon intermediates.

*CsrA* modulates three enzymes that participate directly in PEP metabolism; as shown in Fig. 1, PykF is positively-regulated, while PEP carboxykinase (PckA) and PEP synthetase (Pps), which generate PEP from oxaloacetate and pyruvate, respectively, are negatively regulated. Several enzymes that indirectly affect PEP are also regulated by CsrA. Thus, a *csrA* mutation causes a significant elevation in intracellular PEP pools. However, notably, *csrA* exhibits little or no effect on two genes of the pentose phosphate pathway, which generates E4P.

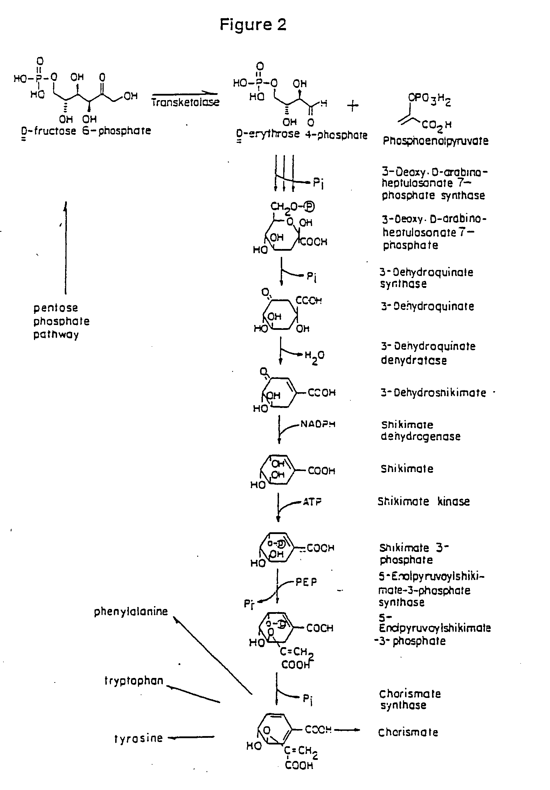

As shown in Fig. 2, PEP, a precursor of aromatic amino acids (tyrosine, phenylalanine, and tryptophan) and other metabolic products, combines with E4P in a first condensation reaction, to produce DAHP in a reaction catalyzed by DAHP synthase. DAHP is in turn converted to 3-dehydroquinate, then to 3-dehydroshikimate, then to shikimate, then to shikimate-3-phosphate by shikimate kinase and then to a EPSP in a second condensation reaction with PEP and finally to chorismate.

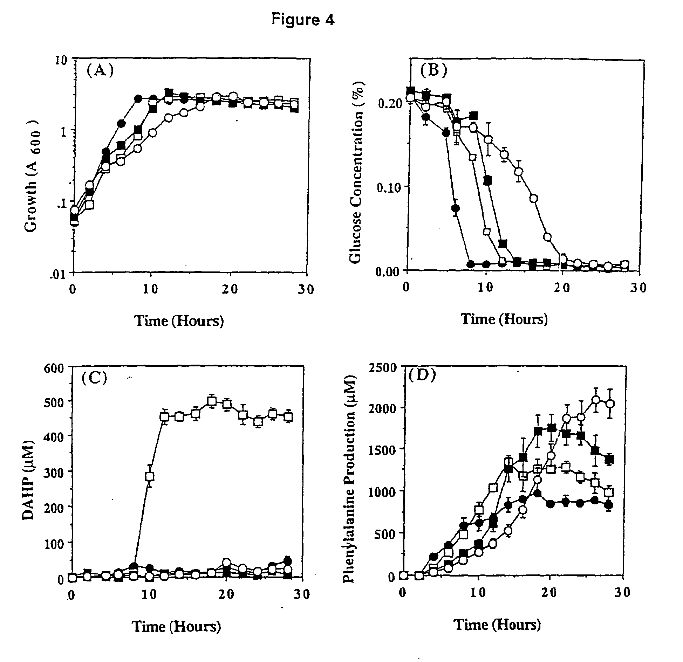

As described more fully below, despite an increase in E4P engineered by introducing the plasmid pAT1 (including the transketolase gene) into the strain NST74 (ATCC No. 31884, see U.S. Patent No.4,681,852 - the'852 patent) engineered for optimal phenylalanine production (including the tyrR gene mutation which increase the expression of, inter alia, DAHP synthase and shikimate kinase), the amount of PEP produced by the TR1-5 mutation was sufficient for both the first and second condensation reactions resulting in 2.2-2.4 fold increase in phenylalanine production. As more fully discussed below, and shown in Fig. 4, the lack of build-up of DAHP suggests that the intracellular PEP in the *csrA* mutants was sufficient for complete conversion of endogenous DAHP to EPSP and ultimately to phenylalanine.

The increase in phenylalanine production in the strains harboring the *csrA* mutation could be due to either an increase in the intrinsic action of enzymes in the pathway leading to phenylalanine production. Two key enzymes in the biosynthesis of phenylalanine, DAHP synthase and chorismate mutase, were test for their activity. These showed no increase in activity in *csrA* mutants (Fig. 2). This finding supports the hypothesis that the increase in phenylalanine production was due to increases in precursor metabolites derived from central carbon metabolism.

As discussed below, and shown in Table 1, the intermediate DAHP, which routinely is used as the measure of flux commitment to the aromatic pathway, as well as phenylalanine production and glucose uptake throughout the growth curves of various strains was tested. Both the *csrA* mutation and pAT1 decreased the growth rate, which was reflected in the kinetics of both glucose uptake and phenylalanine accumulation. The doubling times of NST, NST[pAT1], NST CSR, and NST CSR[pAT1] in a defined medium (MOPS) were approximately 1.38, 1.85, 2.08 and 3.35 hours, respectively. Nevertheless, the final yield of protein in these strains was unaffected, and after 28 hours in culture, 970±51, 954±67, 974±70, and 1024±34 µg/ml of total protein was present, respectively.

As shown in Fig. 4D, the production of phenylalanine over time in a batch cultures of *E. coli* into which the *csrA* mutation or pAT1 or both had been introduced is markedly different. The plasmid, pAT1, which encoded transketolase, tktA+Cm^{r} and had two tandem lac promoters aroG did increase the production of phenylalanine to 119% (160 mg/L) over the parent strain, but required the additional mutation in *csrA* for maximal amounts 250% (338 mg/L). A disruption of *csrA(csrA::kanR)* alone in an NST lead to improved production by 167% (226 mg/L). As shown in Fig. 4A, phenylalanine production was found to occur during the growth of the organisms and was not found to be a secondary or idophasic metabolite. Maximal rates of phenylalanine production were reached shortly after the termination of the log phase.

In sum, the *csrA* mutant produced almost twice as much phenylalanine as the parent strain; effects of pAT1 were somewhat more modest (~1.4-fold); while the combined effects were approximately additive (~2.2 to 2.4-fold). In these strains, phenylalanine accumulation reached its highest rate in mid- to late-exponential phase of growth and continued for a few hours in the stationary phase.

Mutants of NST glgC and NST both yielded an average of 218 mg/L phenylalanine. Double mutants that contained both the glgC disruption and a *csrA* mutation yielded 139.5mg/L compared to a yield of 288 mg/L in NSTCSR.

As shown in Fig. 4A. the growth of the was affected by the of the csrA mutation (*csrA::kanR*) and pAT1. This was also reflected in the rate of production of protein and glucose utilization. The average generation times under these conditions were 1.38 hours for NST, 1.85 hours for NSTpAT1, 2.08 hours for NSTCsrA, and 3.35 hours for NSTCSRpAT1. The combination of the csrA mutation and the plasmid pAT1 gave the slowest growth and thus it appears that these two mutation are a metabolic drain on the bacteria. Even though NSTCSR and NSTCSRpAT1 had the slowest growth rates they also had the highest rate of phenylalanine production.

As shown in Fig. 4C, DAHP production was not an indication or predictor of phenylalanine production. The cells that had the best DAHP production were those that contained only the pAT1 plasmid. High levels of DAHP have been also observed (Patnaik and Liao (1994); and Gosset et al. (1996)) in a different strain background, but with the same plasmid. In the wild type bacteria, NST, the production of DAHP is balanced with growth requirements. The introduction of plasmid pAT1 which encodes for transketolase and AroGfbr would be expected to cause increases flux of carbon toward the formation of DAHP and towards its accumulation as is seen with NSTpAT1. The disruption of the *csrA* gene eliminated this accumulation of DAHP and instead favored production of phenylalanine.

The precursors of the shikimate pathway are a single E4P and two PEP molecules. The Km for PEP was reported to be ~5.8 µM for DAHP synthases (AroF and AroG), but was significantly higher for EPSP synthase, 16 µM. Pittard (1996) Cell. Mol. Biol. Vol. 2 2^{nd} edn. Neidhardt et al. (eds). American Society for Microbiology, Washington, D.C. Thus the increased intracellular PEP in the *csrA* mutants was sufficient for complete conversion of endogenous DAHP to EPSP and ultimately to phenylalanine. These results demonstrate a potential pitfall of measuring a single intermediate as an indication of pathway flux in metabolically-engineered strains, as is often conducted. Commitment of precursor to a biosynthetic pathway is not necessarily equivalent to flux through the pathway and into the desired end product.

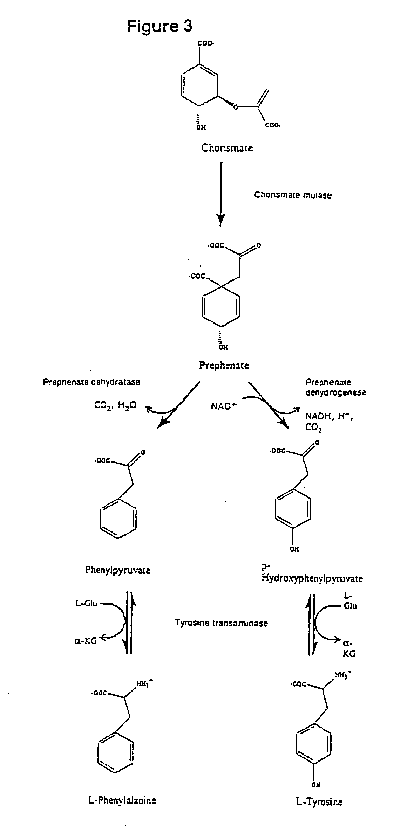

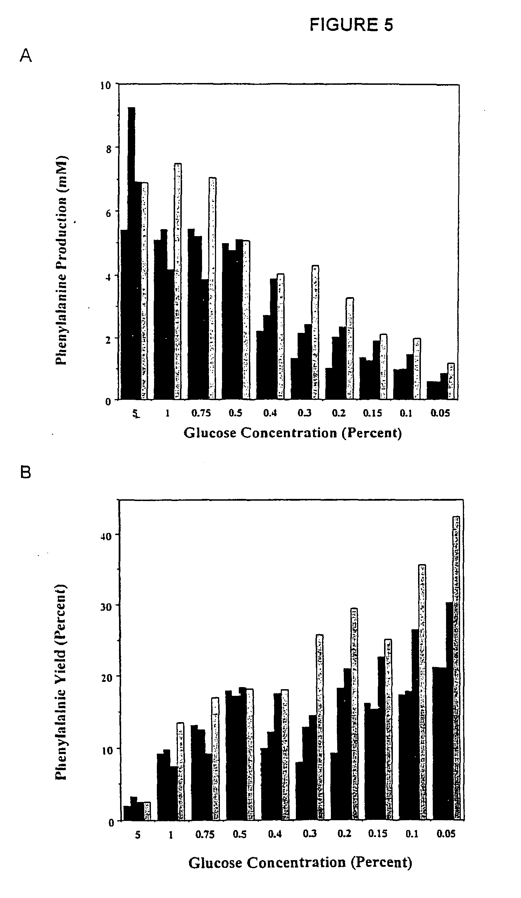

As shown in Fig. 5, the production of phenylalanine was affected by carbon. Glucose concentrations up to 0.4 % yielded a highly consistent pattern of relative productivity for the strains: NST<NST[pAT1]<NST CSR<NST CSR[pAT1]. Beyond 0.4 % glucose, productivity increased in each of the strains, but the pattern was less regular. Phenylalanine synthesis in these strains tended to out-compete biomass production as carbon substrate became limiting. At carbon loading great than one, the effects of both disruption of the *csrA* gene and the addition of plasmid pAT1 can be seen in the amounts of phenylalanine production. The total mass of phenylalanine produced was greater at the higher concentrations of glucose. Theoretical considerations indicate that non-growing *E. coli* can synthesize maximally 3 mol DHAP / 7 mol of glucose (0.43) without recycling pyruvate to PEP, or 6 mol / 7 mol glucose (0.86) with recycling. Patnaik and Liao (1994) Appl. Environ. Microbiol. 60:3903-3908; and Patnaik et al. (1995) Biotech. Bioeng. 46:361-370. Phenylalanine synthesis requires an additional PEP, and the maximum theoretical yield drops to 3 mol / 10 mol glucose, or 6 mol / 10 mol glucose with pyruvate recycling. Substrate conversion to phenylalanine or yield reached ~43% on a weight basis in NST CSR[pAT1] at the lowest concentration of glucose (Fig. 3B) or ~0.39 mol phenylalanine / mol of glucose. Thus, the maximal yield of phenylalanine observed in these growing (i.e. biomass producing) cultures was greater than the theoretical maximum obtainable in a non-growing culture (i.e. a biocatalyst) without pyruvate recycling.

The efficiency of phenylalanine production over a range of glucose concentration is shown in Fig. 2B. There appears to be an optimal concentration between 0.3% and 0.2% of glucose. This is very close to the value, 0.375%, at which glucose would be expected to become the growth limiting substrate. The most efficient phenylalanine production occurs under conditions in which glucose is the growth limiting substrate.

Although the regulatory enzymes DAHP synthase and chorismate mutase are overproduced and are insensitive to feedback inhibition in the NST strain background, it was conceivable that the enhanced phenylalanine production in the *csrA* mutants in part may be caused by a further increase in these regulatory enzymes. As shown in Table 1 below, in fact, the specific activities of these enzymes were modestly lower in *csrA* mutants of both NST and NST[pAT1] and in *csrA* mutants of prototrophic strains of *E. coli* (e.g. MG1655). Thus, these enzymes were not restricting aromatic flux in the NST strains.

**Table 1. Regulatory enzymes of phenylalanine biosynthesis.**

| Relative specific activity (% NST±S.D.) | | |
|---|---|---|
| Strain | Chorismate mutase^{a} | DAHP synthase |
| NST[pAT1] | 120±17 | 122±17 |
| NST CSR | 66±11 | 34±12 |
| NST CSR[pAT1] | 46±12 | 49±17 |

| | | |
|---|---|---|
| ^{a}Specific activities of chorismate mutase and DAHP synthase in NST were 447±70 and 103±15 µmol/min mg protein, respectively. Relative specific activities of chorismate mutase and DAHP synthase were averaged for 3 and 4 independent determinations, respectively. | | |

In order to further optimize carbon flow into the desired products, engineering of gluconeogenesis, glycogen biosynthesis and possibly other pathways are desirable. A mutation in *fbp*, which encodes fructose-1,6-bisphosphatase, would theoretically prevent gluconeogenesis from proceeding beyond the synthesis of fructose-1,6-bisphosphate. A mutation in *glgC* (ADP-glucose pyrophosphorylase) or *glgA* (glycogen synthase) prevents residual glucose derivatives obtained from the media or generated within the cell from being used for glycogen synthesis. Each of these mutations are already known and could be introduced into a cell either by P1 transduction from existing mutants or new mutations can be generated in appropriate commercial *E. coli* strains. The synthesis of a desired amino acid or other aromatic compound could be further enhanced by introducing mutations which block the synthesis of one or two of the three aromatic acids assuming that growth requirements are met by the growth medium.

The hypothesis that the production of a desired product of a biosynthetic pathway not directly regulated by *csrA* can be increased by blocking the carbon flux into a competing pathway which is controlled by *csrA* was tested under one set of growth conditions for phenylalanine production.

The *csrA* mutation diverts carbon flux into glycogen synthesis, which may compete with aromatic amino acid production. However, disruption of ADPglucose pyrophosphoryiase and glycogen synthase genes (*glgCA*) with a polar transposon insertion did not affect phenylalanine production in NST CSR and was somewhat inhibitory in NST CSR[pAT1]. These results are explained by the observation that the NST strains, including the *csrA* mutants, synthesized little or no glycogen on the modified MOPS medium that was used for phenylalanine determinations. However, they accumulated glycogen under CsrA control on Kornberg agar, and prototrophic strains of *E. coli* accumulated glycogen on the modified MOPS medium. Apparently, the glycogen biosynthesis pathway cannot compete effectively with the shikimate pathway on MOPS medium in these engineered strains.

Although genetic regulation has been exploited in metabolic engineering schemes to our knowledge, this is the first example utilizing a global regulatory pathway. Because global regulatory networks coordinate virtually all of the metabolic activities and developmental pathways of the cell, these powerful and easily manipulated systems should be attractive targets for strain improvement. In our approach, an effector protein was disrupted to alter global control. However, sensor proteins, ligands, antagonists (e.g. CsrB) or other molecules affecting regulation could be targeted. Over-expression of a regulator or modification of its specificity could also provide a means of altering global control. Thus, it should be feasible to either elicit or block major physiological responses or developmental shifts that affect product yields in various species (e.g. responses to nutrient starvation, anaerobiosis, population density, or sporulation of gram positive bacteria). Because global regulators modulate numerous steps of metabolism, the genetic engineering of a single global regulatory gene may alter many or all of the reactions of a useful pathway. This should avoid certain caveats of approaches that focus on specific enzymes. For example, it is not uncommon to find that multiple steps of a given pathway are flux-determining, or that the increased expression of a rate-limiting enzyme simply shifts the flux control to other steps of the pathway. Finally, CsrA and certain other global regulators exert opposite effects on opposing metabolic pathways making it possible to simultaneously enhance a desired pathway and inhibit competitive pathways by engineering a single gene. Sabnis et al. (1995) J. Biol. Chem. 270:29096-29104; and Saier et al. (1996) Cell. Molec. Biol. Vol.2, 2^{nd} edn. Neidhardt et al. (eds) American Society for Microbiology, Washington, D.C.

Various components of NST74 will now be described with reference to Figs. 2 and 3. As explained more fully described in the '852 patent the increased phenylalanine production occurs if the presence of high levels of feedback inhibition desensitized DAHP synthase is combined with removal of both repression and inhibition controls on at least the first step in the phenylalanine terminal pathways. It will be understood by persons skilled in the art that inhibition of an enzyme refers to the diminution of the activity of the enzyme while repression of the formation of an enzyme refers to a diminution in the rate at which the enzyme is synthesized by the microorganism.

The wild type *E. coli* produces DAHP synthase in at least three forms which are respectively subject to feedback inhibition by phenylalanine, tyrosine or tryptophan. It has been found that the genetic constitution of the microorganism can be altered such that at least one form of the enzyme i.e. one isoenzyme is produced which is not subject to feedback inhibition by any of the three above-mentioned amino acids. It is preferred that feedback inhibition by both phenylalanine and tyrosine be removed, which can be achieved by mutation of their respective structural genes aroG and aroF to yield genes aroG(FBI®) and aroF(FBI®) respectively.

The formation of the various forms of DAHP synthase is subject to repression in the wild type bacterium as a result of the production by the microorganism of a regulatory protein which is a product of a control, or regulatory gene tyrR. This repression may be overcome by a mutation of the gene tyrR to remove the repression. It is obvious to those skilled in the art that the elevation of enzyme levels that is obtained by using tyrR mutants may be obtained readily by alternative approaches. One example is by the use of multi-copy plasmids having the appropriate structural genes for the production of the enzyme. If the latter method is used the repression would still exist but the enzyme levels can be raised to a satisfactory level by having a multiplicity of structural genes each of which is active in the formation of the desired enzyme. Alternatively, the open coding region of the gene maybe expressed from a heterologous promoter that is not regulated by TyrR.

The gene tyrR controls production of two forms of DAHP synthase, including DAHP synthase Phe and DAHP synthase Tyr. It is preferable that any mutation of control gene tyrR should have the effect of removing repression on these two forms of DAHP synthase.

A desired microorganism can have a mutant form of tyrR that removes repression of the production of one of the forms of DAHP synthase such as the production of DAHP synthase Phe or multi-copy plasmids containing the structural genes for the production of the other forms of the enzyme, such as plasmids containing the gene aroF which encodes DAHP synthase Tyr (or other known mechanism of increased expression of the gene, e.g. via promoter etc.).

As shown in Fig. 3, wild type *E. coli* produces the enzyme chorismate mutase P-prephenate dehydratase (CMP-PDH) which catalysts the first two steps in the terminal pathway producing phenylalanine from chorismate. This enzyme in the wild type microorganism is subject to inhibition by phenylalanine. This enzyme is encoded by the structural gene pheA and mutation of this gene to pheA (FBI®) enables the organism to produce the enzyme in a form that is not subject to this feedback inhibition by phenylalanine.

The formation of CMP-PDH is subject to repression in the wild type *E. coli* due to the presence of control sequence pheAo. This inhibition may be overcome as has been explained above by mutation of pheAo or, for example, by the use of multi-copy plasmids bearing pheA genes. An example of such a plasmid would be a derivative of the plasmid ColE 1.

The materials erythrose 4-phosphate and (E4P) phosphoenolpyruvate (PEP) and the intermediate shikimate are precursor metabolites for the production of chorismate. A further intermediate in chorismate formation is shikimate-3-phosphate, which is formed from shikimate in a reaction catalyzed by the enzyme shikimate kinase. The production of the enzyme shikimate kinase is subject to repression involving the product of the gene tyrR in the manner outlined above with respect to the repression of the formation of DAHP synthase Phe and DAHP synthase Tyr. This repression can be overcome either by a disruptive mutation in tyrR, with or without a simultaneous effect on the repression of one or more of DAHP synthase isoenzymes, or, for example, by the use of multi-copy plasmids.

In preferred embodiments of this invention related to phenylalanine production, the microorganism according to this invention may be further modified to block the metabolic pathways which lead from chorismate to end products other than phenylalanine. A mutation in the gene tyrA can block the production of tyrosine from chorismate while a mutation in trpE can block the production of tryptophan.

The increased availability of PEP attributable to the disruption of the csrA gene can be used in combination with a genetic element that increases E4P production. The resulting engineered microorganism is further optimized for carbon flow through to EPSP and chorismate (PATH A) or carbon flow through an earlier branching point preceding EPSP (PATH B). See, Fig. 1. For example, 3-dehydroshikimate; 3-dehydroshikimate is the branching point for carbon flow to protocatechuate for catechol or related products. In the latter case, excess availability of PEP, otherwise used in a second condensation with shikimate-3-phosphate, is captured by further increasing E4P and DAHP production. For example, through increasing the expression of transketolase and DAHP synthase, respectively, and other pertinent enzymes, as necessary, e.g. 3-hydroquinate synthase and 3-dehydroquinate dehydratase.

In this context, depending on the branching point to Path B, it would not be necessary or desirable to derepress shikimate kinase as in the case of Path A (flux through EPSP). Mechanisms of blocking the pathway could be implemented. For example, the AroE gene could be mutated in a manner known to the art to block flux beyond 3-dehydroshikimate and growing the microorganism in a complex media containing aromatic amino acids. It will be appreciated, due the comparatively lower Km of EPSP synthase relative to DAHP synthase, that an increased amount of PEP would naturally flow to DAHP and would not be radically influenced by the repression or depression of shikimate kinase. Nevertheless, an optimized strategy that does not imply specific blocks in the pathway can have implications for the choice of implementing tyrR mutants insofar as they impact on shikimate kinase derepression. By contrast, elimination of feedback inhibition and repression of DAHP synthase would be important for progression through either PATH A or B.

Accordingly, Paths A and B define alternative approaches to harvesting the excess PEP, either in the formation of EPSP or in the formation of even greater amounts of DAHP and its derivatives preceding EPSP. For practical application of PATH B, reference is specifically made by way of example, to U.S. Patent No. 5,798,236 related to the production of quinic acid which discloses carbon flux through dehydroquinate only, the shikimate pathway being blocked by eliminating or inhibiting dehydroquinate dehydratase. In this connection, reference is made to U.S. Patent Nos. 5,272,07, 5,847,987, 5,616,496, and 5,629,181 related to production of various products such as catechol, adipic acid and quinic acid. With regard to Path A, reference is also made to U.S. Patent No. 5,939,295 related to the production of tryptophan.

In a preferred embodiment of the invention related to EPSP production leading to chorismate is desirable to employ the mutations of tyrR and *aroF* and preferably both of the AroF (FBI®) AroG (FBI ®) mutants. Alternatively, in place of a tyrR mutation, for example, multicopy plasmids for both DAHP synthase and shikimate kinase could be employed. For phenylalanine production mutations in *pheA* and pheAo are additionally desirable. Accordingly, NST74 is preferred for phenylalanine production.

The TR1-5 mutation or any other mutations that inactivate or down-regulate csrA may be used to increase PEP levels, resulting in increased synthesis of the desired end-products. Other ways of down-regulating csrA, include, for example, regulation by the csrB gene as more fully described in EP application No. 98933282.0; and Romeo (1998) Mol. Microbiol. 29:1321-1330, and antisense nucleic acids capable of specifically binding to *csrA* nucleotide sequences. See, e.g.. EPO publication 431523A2.

### Suitable Culture Conditions

The production of phenylalanine (see methods described in Example 1) hereafter described in example 1 varied with carbon substrate availability.

Suitable carbon sources and other nutritional requirements may be limited by the genotype of the host organism. Regimens for evaluation and optimizing suitable culture conditions are well known to those skilled in the art.

### General Descriptions of Techniques

Unless otherwise stated, the making and use of the invention described herein employs techniques, well known to those skilled in the relevant arts and /or well described in handbooks, treatises and other source books readily available to and understood by those persons.

Techniques for nucleic acid manipulation are described generally, for example, in Sambrook et al. (1989) or Ausubel et al. (1987). Reagents useful in applying such techniques, such as restriction enzymes and the like, are widely known in the art and commercially available from vendors including, but not limited to, New England BioLabs, Boehringer Mannheim, Amersham, Promega Biotec, U.S. Biochemicals, New England Nuclear, and a number of other sources.

### Probes, primers, and antisense nucleic acids

Nucleic acid probes and primers based on csrA sequences may be prepared by standard techniques. Such a probe or primer comprises an isolated nucleic acid. In the case of probes, the nucleic acid further comprises a label (e.g., a radionuclide such as ³²P or ³⁵S) or a reporter molecule (e.g., a ligand such as biotin or an enzyme such as horseradish peroxidase). Probes may be used to identify the presence of a hybridizing nucleic acid sequence, e.g., a csrA mRNA in a sample or a cDNA or genomic clone in a library. Primers may be used, for example, for amplification of nucleic acid sequences, e.g.. by the polymerase chain reaction (PCR). See, e.g., PCR Protocols: A Guide to Methods and Applications, Innis, M., et al., eds.. Academic Press: San Diego (1990). The preparation and use of probes and primers is described, e.g., in Sambrook et al. (1989) or Ausubel et al. (1987).

Nucleic acids may be produced in large amounts by replication of a suitable recombinant vector in a compatible host cell. Alternatively, these nucleic acids may be chemically synthesized, e.g., by any method known in the art, including, but not limited to, the phosphoramidite method described by Beaucage and Carruthers (1981) Tetra. Letts. 22:1859-1862 and the triester method according to Matteucci et al. (1981) J. Am. Chem. Soc. 103:3185, preferably using commercial automated synthesizers.

DNA constructs prepared for introduction into a prokaryotic or eukaryotic host typically comprise a replication system recognized by the host, including the intended DNA fragment encoding the desired polypeptide, and preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, transcriptional terminator sequences, and mRNA stabilizing sequences. Secretion signals from secreted polypeptides may also be included to allow the polypeptide to cross and/or lodge in cell membranes or be secreted from the cell. Such vectors may be prepared by means of standard recombinant techniques discussed, for example, in Sambrook et al. (1989) or Ausubel et al. (1987).

An appropriate promoter and other necessary vector sequences are selected so as to be functional in the host, and may, when appropriate, include those naturally associated with *csrA* genes. Examples of workable combinations of cell lines and expression vectors are described in Sambrook et al. (1989) or Ausubel et al. (1987); see also, e.g., Metzger et al. (1988) Nature 334:31-36. Many useful vectors are known in the art and may be obtained from vendors including, but not limited to, Stratagene, New England BioLabs, Promega Biotech, and others.

Promoters including but not limited to, the trp, lac and phage promoters, tRNA promoters and glycolytic enzyme promoters may be used in prokaryotic hosts. While expression vectors are preferably autonomously replicating, they may also be inserted into the genome of the host cell by methods known in the art. Expression and cloning vectors preferably contain a selectable marker which is a gene encoding a protein necessary for the survival or growth of a host cell transformed with the vector. The presence of this gene ensures the growth of only those host cells that express the inserts. Typical selection genes are known in the art and include, but are not limited to, those which encode proteins that (a) confer resistance to antibiotics or other toxic substances, e.g. ampicillin, tetracycline, kanamycin, chloramphenicol etc.; (b) complement auxotrophic deficiencies, or (c) supply critical nutrients not available from complex media, e.g. the gene encoding D-alanine racemase for Bacilli. The choice of the proper selectable marker depends on the host cell, and appropriate markers for different hosts are well known.

The genetic elements of the present invention can be introduced into host cells by any method known in the art. Such elements vary depending on the type of cellular host, and include, but are not limited to, electroporation; transfection employing calcium chloride, rubidium chloride calcium phosphate, DEAE-dextran, or other substances; microprojectile bombardment; lipofection; infection (where the vector is an infectious agent, such as a retroviral genome); and other methods. See: generally, Sambrook et al. (1989) and Ausubel et al. (1987). The cells into which these nucleic acids have been introduced also include the progeny of such cells.

Mutations maybe transferred from one bacterial strain to another by transduction using e.g. plvir as described for csrA::kanR in the '144 patent.

The following examples are provided to illustrate, but not limit, the invention.

### Example 1

### Increased Phenylalanine Production

The *E. coli* K-12 strain NST 74 (ATCC 31884) or NST had the following relevant genotype: *aroF394 aroG397 tryR366 pheA101 pheO352 malT384*. U.S. Patent No. 4,681,852. The designation CSR indicates that the wild type *csrA* allele was replaced with *csrA::kanR* using P1*vir* transduction. Romeo et al. (1993) J. Bacteriol. 175:4744-4755; and the '144 patent. The plasmid pAT1 contained *tktA*, encoding transketolase, and *aroG*^{*fhr*} expressed via two tandem *lac* promoters and was a generous gift of James C. Liao (University of California, Los Angeles). U.S. Patent Nos. 5,906,925; 5,985,617; 5,168,056; and 5,776,736; Patnaik et al. (1994) Appl. Environ. Microbiol. 60:3903; and Patnaik et al. Biotech. Bioeng. 46:361-370.

Overnight cultures were grown in LB medium. Miller (1972) Experiments in Molecular Genetics. Cold Spring Harbor Laboratory, Cold Spring Harbor, N.Y. For all experiments, overnight cultures were used to inoculate (at 1:100 dilution) a chemically-defined medium, MOPS(12), which was modified to contain 1.32 mM KH₂PO₄, 7.125 mM NH₄Cl, 2 mg/L thiamine and 0.2% glucose, unless otherwise indicated. Growth in this medium should be limited by phosphate availability. Batch cultures were grown at 37°C with gyratory agitation at 250 rpm. Strains containing pAT1 were grown under antibiotic selection with chloramphenicol at 20 µg/ml. Growth of colonies for detection of endogenous glycogen was on Kornberg medium. Liu et al. (1995) J. Bacteriol. 177:2663-2672.

At the indicated time points, cultures for determination of phenylalanine production or glucose utilization were centrifuged to remove cells and the supernatant solutions were either assayed immediately stored frozen (-20°C). Determination of glucose was by the method of Dubois et al. (1956) Anal. Chem. 18: 350-356 and phenylalanine was assayed by HPLC. Samples to be analyzed for total protein and DAHP were treated with trichloroacetic acid (TCA) to a final concentration of 10% and stored frozen (-20°C), pending analysis. Protein in the TCA precipitates was collected by centrifugation and quantified by the bicinchoninic acid procedure using bovine serum albumin as a standard. Smith et al. (1985) Anal. Biochem. 150:76-85. DAHP in the supernatant solution was assayed by the thiobarbituric acid method. Doy and Brown (1965) Biochem. Biophys. Acta 104:377-389. Semi-quantitative staining of glycogen in colonies with iodine vapor was conducted as previously described. Liu et al. (1995). Analytical HPLC was performed on a Rainin Rabbit system equipped with a Knauer UV-visible detector (Rainin Instrument Co, Woburn, MA). Separation of phenylalanine was conducted using a Supelco Sil LC 18 column (4.6 X 250 mm) (Supelco Inc., Bellefonte, PA) with isocratic elution at 1.0 ml/min, in 40% methanol in water, containing 0.2% triflouroacetic acid.

Cells for enzyme analysis were harvested from 1 L cultures by centrifugation at mid-exponential phase of growth and stored at -80°C. Cells were thawed, lysed by sonication in at least 4-fold excess of phosphate buffer (100 mM, pH 6.4), and cell debris was removed by centrifugation. The cell-free preparation was assayed for total chorismate mutase (Dopheide et al. (1972) J. Biol. Chem. 247:4447-4452) and DAHP synthase (Doy and Brown (1965)) activities. Values for enzyme activities were determined within the linear range with respect to the amount of cell extract added.

Phosphoenolpyruvate (PEP) concentration was determined by the method described by Lamprecht and Heinz (1988) In, Methods of Enzymatic Analysis, Vol. VI. Bergmeyer et al. Eds. VCH Verlagsgeselischaft, Weinheim, Federal Republic of Germany pp.555-561. Bacterial paste (0.25 ml) was extracted by sonication in the presence of 1.0 M perchloric acid (0.5ml), the soluble fraction was collected by centrifugation and the procedure repeated this time with 0.25 ml perchloric acid. The total volume of each extract was 1.0 ml. The total soluble extracts were neutralized with a minimal amount of potassium carbonate and 0.5 ml was assayed for PEP using a coupled reaction of pyruvate kinase followed by lactate dehydrogenase. The assay measured the decrease in absorbance at 339nm due to the oxidation of NADH (Molar=6290).

Although the foregoing invention has been described in some detail by way of illustration and example for purposes of clarity and understanding, it will be apparent to those skilled in the art that certain modifications may be practiced. Therefore, the description and examples should not be construed as limiting the scope of the invention, which is delineated by the appended claims.

## Claims

1. A microorganism comprising a first genetic element which decreases the available amount of functional CsrA protein and a second genetic element which increases the amount of D-erythrose-4-phosphate (E4P) wherein the microorganism is genetically adapted to produce, under suitable nutrient supply, amounts of phosphoenolpyruvate (PEP), sufficient to convert E4P to 3-deoxy-D-arabino-heptulosonate-7-phosphate (DAHP) and resulting shikimate-3-phosphate to 5-enolpyruvoylshikimate-3-phosphate (EPSP).

2. A microorganism according to claim 1 which is genetically adapted to produce an amount of PEP sufficient to convert E4P to DAHP and resulting shikimate-3-phosphate to EPSP under conditions of increased production or non-limiting amounts of DAHP synthase and other enzymes participating in synthesis of compounds which are intermediate in the pathway between DAHP and EPSP.

3. A microorganism according to claim 1 or 2 belonging to the species *E. coli*.

4. A microorganism according to any preceding claim wherein the second genetic element is a plasmid which encodes the transketolase gene (tktA).

5. A microorganism according to any preceding claim comprising an additional genetic element which feedback derepresses an isozyme of DAHP synthase and/or which increases the expression of DAHP synthase.

6. A microorganism according to any preceding claim comprising a first additional genetic element which increases the amount of shikimate kinase.

7. A microorganism according to claim 5 or 6 wherein the additional genetic element is AroF (FB1), AroG (FB1) or a *tyrR* mutant.

8. A microorganism according to any preceding claim wherein the nutrient supply comprises glucose as a primary carbon source or has glucose present in an amount between 0.2% and 0.4%.

9. A microorganism according to any preceding claim comprising a second additional genetic element which derepresses chorismate mutase P-prephenate dehydratase (CMP-PDH).

10. A microorganism according to claim 6 or 9 which feedback derepresses CMP-PDH.

11. A microorganism according to claim 9 or 10 wherein the second additional genetic element is a pheAo mutant or pheA (FBI).

12. A microorganism according to any preceding claim which is genetically adapted from the strain NST74 designated ATCC 31884, the mutant strain of *E. coli* NST 37 and designated ATCC 31882 or the mutant strain of E. *coli* NST 70 and designated ATCC 31883.

13. A microorganism according to claim 1, which is a strain of *E. coli* having altered production of 3-deoxy-D-arabino-heptulosonate-7-phosphate (DAHP) synthase and chorismate mutase P-prephenate dehydratase (CMP-PDH), wherein
i) the DAHP synthase is not subject to feedback inhibition by phenylalanine, tyrosine, or tryptophan present within the microorganism and the DAHP synthase either
(a) is not subject to repression by phenylalanine, tyrosine, or tryptophan; and
(b) is produced in greater amount than is produced by wild type *E. coli;* and
ii) the CMP-PDH is not subject to feedback inhibition by phenylalanine and the CMP-PDH either
(a) is not subject to repression by phenylalanine; or
(b) is produced in a greater amount than is produced by wild type *E*. *coli.*

14. A microorganism according to claim 1, which is a strain of *E. coli* having altered production of shikimate kinase wherein
i) the shikimate kinase is not repressed by phenylalanine, tyrosine or tryptophan; or
ii) the amount of said shikimate kinase produced is greater than that produced by a wild-type *E. coli*.

15. A microorganism according to claim 1, which is a strain of *E. coli* and in which:
a) the DNA of the microorganism is engineered such that DAHP synthase of the microorganism is desensitized to inhibition by both phenylalanine and tyrosine;
b) the formation of DAHP synthase in the microorganism is desensitized to repression by mutation of the control gene *tyrR* of the microorganism; and/or
c) the metabolic pathways of the microorganism are blocked by mutation of the gene tyrA to block production of tyrosine or mutation of the gene *trpE* to block production of tryptophan.

16. A microorganism according to claim 1, 5 or 12 which is a strain of *E. coli* comprising multi-copy plasmids, each of the multi-copy plasmids having a structural gene for the production of at least one member of the group consisting of DAHP synthase, CMP-PDH and shikimate kinase.

17. A microorganism genetically adapted to produce an increased amount of product constituted from carbon resources flowing through the shikimate pathway through EPSP, the microorganism comprising genetic elements selected from at least the following:
a) a first genetic element which decreases the amount of functional CsrA protein;
b) a second genetic element which increases the amount of E4P;
c) one or more additional genetic elements whose feedback derepresses one or more isozymes of DAHP synthase;
d) one or more additional genetic elements which increases the amount of DAHP synthase;
e) one or more additional genetic elements which increases the amount of shikimate kinase; and
f) a mutation in the tyrR gene;
wherein the microorganism comprises at least the first and second genetic elements.

18. A microorganism that is genetically adapted to produce enhanced amounts of compounds flowing through the common aromatic pathway to a target compound which is intermediate in the pathway between DAHP and EPSP, the microorganism comprising a first genetic element which decreases the amount of functional CsrA protein and a second genetic element which increases the amount of E4P, whereby the microorganism is adapted to channel excess PEP into the production of the target compound under conditions of increased or non-limiting amounts of E4P, DAHP synthase and preferably other enzymes within the pathway leading to the target compound.

19. A method for the production of phenylalanine comprising the steps of culturing a strain of *E. coli* in a medium containing a carbon source and recovering the phenylalanine, wherein the strain of *E. coli* has:
a. a first genetic element which decreases CsrA protein;
b. a second genetic element which increases D-erythrose-4-phosphate (E4P); and
c. altered production of 3-deoxy-D-arabino-heptulosonate-7-phosphate (DAHP) synthase and chorismate mutase P-prephenate dehydratase (CMP-PDH), wherein
i) the DAHP synthase is not subject to feedback inhibition of phenylalanine, tyrosine, or tryptophan present with the microorganism and the DAHP synthase either;
(a) is not subject to repression by phenylalanine, tyrosine or tryptophan; or
(b) is produced in greater amount than is produced by wild type *E. coli*; and
ii) the CMP-PDH is not subject to feedback inhibition by phenylalanine and the CMP-PDH either:
(a) is not subject to repression by phenylalanine; or
(b) is produced in a greater amount than is produced by wild type *E. coli*.

20. A method according to claim 19 in which the carbon source comprises glucose, glycerol, xylose, maltose, lactose, lactate or acetic acid, or the DNA microorganism has been further modified to prevent the production from chorismate of end products other than phenylalanine.

21. A method of producing an increased amount of metabolic products of the shikimate pathway including EPSP and preferably chorismate by culturing, under suitable culture conditions, particularly suitable nutrient conditions, a micro-organism comprising a genetic element which decreases the amount of available csrA gene product and a genetic element which causes an increase in the amount of E4P, whereby said microorganism is genetically adapted to produce sufficient amounts of PEP to convert E4P to DAHP and to convert resulting shikimate-3-phosphate to EPSP.

22. A method of producing enhanced amounts of compounds flowing through the common pathway to a target compound which is intermediate in the pathway between DAHP and EPSP by culturing a microorganism under suitable nutrient conditions a microorganism comprising a first genetic element which decreases the amount of functional CsrA protein and a second genetic element which increases the amount of E4P, whereby said microorganism is genetically adapted to channel excess PEP into the production of said target compound under conditions of increased production or non-limiting amounts of E4P, DAHP synthase and, optionally, other enzymes within the pathway leading to the target compound.

23. A microorganism which has been genetically adapted to enhance the synthesis of aromatic substances comprising a first genetic element which decreases the available amount of functional CsrA protein and a second genetic element which increases the amount of D-erythrose-4-phosphate (E4P).

## Patentansprüche

1. Mikroorganismus, umfassend ein erstes genetisches Element, welches die verfügbare Menge an funktionellem CsrA-Protein herabsetzt, und ein zweites genetisches Element, welches die Menge an D-Erythrose-4-phosphat (E4P) erhöht, wobei der Mikroorganismus genetisch angepasst ist, um, unter geeigneter Nährstoffzufuhr, ausreichende Mengen an Phosphoenolpyruvat (PEP) zu produzieren, um E4P zu 3-Desoxy-D-arabino-heptulosonat-8-phosphat (DAHP) und das resultierende Shikimat-3-phosphat zu 5-Enolpyruvylshikimat-3-phosphat (EPSP) umzuwandeln.

2. Mikroorganismus nach Anspruch 1, welcher genetisch angepasst ist, um eine ausreichende Menge an PEP zu produzieren, um E4P zu DAHP und das resultierende Shikimat-3-phosphat zu EPSP unter Bedingungen einer erhöhten Produktion oder nicht-beschränkender Mengen an DAHP-Synthase und anderer Enzymen, die in der Synthese von Verbindungen beteiligt sind, die Intermediate im Weg zwischen DAHP und EPSP darstellen, umzuwandeln.

3. Mikroorganismus nach Anspruch 1 oder 2, der zur Spezies *E. coli* gehört.

4. Mikroorganismus nach einem der vorangehenden Ansprüche, wobei das zweite genetische Element ein Plasmid ist, das das Transketolasegen (tktA) codiert.

5. Mikroorganismus nach einem der vorangehenden Ansprüche, umfassend ein zusätzliches genetisches Element, welches ein Isozym der DAHP-Synthase Feedback-dereprimiert und/oder welches die Expression von DAHP-Synthase erhöht.

6. Mikroorganismus nach einem der vorangehenden Ansprüche, umfassend ein erstes zusätzliches genetisches Element, das die Menge an Shikimatkinase erhöht.

7. Mikroorganismus nach Anspruch 5 oder 6, worin das zusätzliche genetische Element AroF (FB1), AroG (FB1) oder eine *tyrR*-Mutante ist.

8. Mikroorganismus nach einem der vorangehenden Ansprüche, wobei die Nährstoffzufuhr Glucose als einer primären Kohlenstoffquelle umfasst oder in welcher Glucose in einer Menge zwischen 0,2 % und 0,4 % vorhanden ist.

9. Mikroorganismus nach einem der vorangehenden Ansprüche, umfassend ein zweites zusätzliches genetisches Element, das Chorismat-Mutase-P-Prephenat-Dehydratase (CMP-PDH) dereprimiert.

10. Mikroorganismus nach Anspruch 6 oder 9, welches CMP-PDH Feedbackdereprimiert.

11. Mikroorganismus nach Anspruch 9 oder 10, worin das zweite zusätzliche genetische Element eine pheAo-Mutante oder pheA (FBI) ist.

12. Mikroorganismus nach einem der vorangehenden Ansprüche, welcher genetisch angepasst ist von dem Stamm NST 74, bezeichnet als ATCC 31884, dem Mutantenstamm von *E. coli* NST 37 und bezeichnet als ATCC 31882 oder dem Mutantenstamm von *E. coli* NST 70 und bezeichnet als ATCC 31883.

13. Mikroorganismus nach Anspruch 1, welcher ein Stamm von *E. coli* mit einer veränderten Produktion von 3-Desoxy-D-arabino-heptulosonat-7-phosphatase (DAHP)-Synthase und Chorismat-Mutase-P-Prephenat-Dehydratase (CMP-PDH) ist, worin
i) die DAHP-Synthase keiner Feedback-Hemmung durch Phenylalanin, Tyrosin oder Tryptophan unterliegt, die innerhalb des Mikroorganismus' vorhanden sind, und die DAHP-Synthase entweder
a) keiner Repression durch Phenylalanin, Tyrosin oder Tryptophan unterliegt; oder
b) in größeren Mengen produziert wird als sie durch Wildtyp-*E. coli* produziert wird; oder
ii) die CMP-PDH keiner Feedback-Hemmung durch Phenylalanin unterliegt und die CMP-PDH entweder
a) keiner Repression durch Phenylalanin unterliegt; oder
b) in größeren Mengen produziert wird als sie durch Wildtyp-*E. coli* produziert wird.

14. Mikroorganismus nach Anspruch 1, welcher ein Stamm von *E. coli* mit einer veränderten Produktion von Shikimatkinase ist, worin
i) die Shikimatkinase nicht durch Phenylalanin, Tyrosin oder Tryptophan reprimiert wird; oder
ii) die Menge an produzierter Shikimatkinase größer ist als die durch ein Wildtyp-*E. coli* produzierte Menge.

15. Mikroorganismus nach Anspruch 1, welcher ein Stamm von *E. coli* ist und worin:
a) die DNA des Mikroorganismus so konstruiert ist, dass die DAHP-Synthase des Mikroorganismus desensibilisiert ist gegenüber der Hemmung sowohl durch Phenylalanin als auch Tyrosin;
b) die Bildung der DAHP-Synthase im Mikroorganismus desensibilisiert ist gegenüber der Repression durch die Mutation des Kontrollgens *tyrR* des Mikroorganismus; und/oder
c) die Stoffwechselwege des Mikroorganismus durch die Mutation des Gens *tyrR* zur Blockierung der Produktion von Tyrosin oder die Mutation des Gens *trpE* zur Blockierung des Produktion von Tryptophan blockiert sind.

16. Mikroorganismus nach Anspruch 1, 5 oder 12, welcher ein Stamm von *E. coli* ist, umfassend multi-copy-Plasmide, wobei jedes der multi-copy-Plasmide ein Strukturgen für die Produktion von wenigstens einem Mitglied der Gruppe, bestehend aus DAHP-Synthase, CMP-PDH und Shikimatkinase, aufweist.

17. Mikroorganismus, der genetisch angepasst ist, um eine erhöhte Menge an Produkt zu produzieren, das aufgebaut ist aus Kohlenstoffquellen, die durch den Shikimatweg über EPSP fließen, welcher Mikroorganismus genetische Elemente umfasst, ausgewählt aus zumindest den folgenden:
a) einem ersten genetischen Element, welches die Menge an funktionellem CsrA-Protein herabsetzt;
b) einem zweiten genetischen Element, welches die Menge an E4P erhöht;
c) einem oder mehreren zusätzlichen genetischen Elementen, deren Feedback ein oder mehrere Isozyme der DAHP-Synthase dereprimiert;
d) einem oder mehreren zusätzlichen genetischen Elementen, welche die Menge an DAHP-Synthase erhöhen;
e) einem oder mehreren zusätzlichen genetischen Elementen, welche die Menge an Shikimatkinase erhöhen; und
f) einer Mutation im *tyrR*-Gen;
wobei der Mikroorganismus wenigstens die ersten und zweiten genetischen Elemente umfasst.

18. Mikroorganismus, der genetisch angepasst ist, um erhöhte Mengen an Verbindungen zu produzieren, die durch den "common aromatic pathway" zu einer Zielverbindung fließen, die ein Intermediat im Weg zwischen DAHP und EPSP ist, welcher Mikroorganismus ein erstes genetisches Element, das die Menge an funktionellem CsrA-Protein herabsetzt, und ein zweites genetisches Element umfasst, welches die Menge an E4P erhöht, wobei der Mikroorganismus zum Kanalisieren von überschüssigem PEP in die Produktion der Zielverbindung unter Bedingungen erhöhter oder nicht-beschränkender Mengen an E4P, DAHP-Synthase und vorzugsweise anderer Enzyme innerhalb des Wegs, der zu der Zielverbindung führt, angepasst ist.

19. Verfahren zur Produktion von Phenylalanin, umfassend die Schritte des Züchtens eines Stamms von *E. coli* in einem Medium, das eine Kohlenstoffquelle enthält, und Rückgewinnens des Phenylalanins, wobei der Stamm von *E. coli* Folgendes aufweist:
a. ein erstes genetisches Element, welches CsrA-Protein vermindert;
b. ein zweites genetisches Element, welches D-Erythrose-4-phosphat (E4P) erhöht; und
c. eine veränderte Produktion von 3-Desoxy-D-arabino-heptulosonat-7-phosphat (DAHP)-Synthase und Chorismat-Mutase-P-Prephenat-Dehydratase (CMP-PDH), worin
i) die DAHP-Synthase keiner Feedback-Hemmung durch Phenylalanin, Tyrosin oder Tryptophan unterliegt, die innerhalb des Mikroorganismus' vorhanden sind, und die DAHP-Synthase entweder
a) keiner Repression durch Phenylalanin, Tyrosin oder Tryptophan unterliegt; oder
b) in größeren Mengen produziert wird als sie durch Wildtyp-*E. coli* produziert wird; und
ii) die CMP-PDH keiner Feedback-Hemmung durch Phenylalanin unterliegt und die CMP-PDH entweder
a) keiner Repression durch Phenylalanin unterliegt; oder
b) in größeren Mengen produziert wird als sie durch Wildtyp-*E. coli* produziert wird.

20. Verfahren nach Anspruch 19, wobei die Kohlenstoffquelle Glucose, Glycerol, Xylose, Maltose, Lactose, Lactat oder Essigsäure umfasst, oder der DNA-Mikroorganismus weiter modifziert worden ist, um die Produktion aus Chorismat von anderen Endprodukten als Phenylalanin zu verhindern.

21. Verfahren zur Produktion einer erhöhten Menge an Stoffwechselprodukten des Shikimatwegs einschließlich EPSP und vorzugsweise Chorismat durch Züchten, unter geeigneten Kulturbedingungen, insbesondere geeigneten Nährstoff-Bedingungen, eines Mikroorganismus, umfassend ein genetisches Element, welches die Menge an verfügbarem csrA-Genprodukt herabsetzt, und ein genetisches Element, welches einen Anstieg der Menge an E4P bewirkt, wobei der Mikroorganismus genetisch angepasst ist, um ausreichende Mengen an PEP zu produzieren, um E4P zu DAHP umzuwandeln und um resultierendes Shikimat-3-phosphat zu EPSP umzuwandeln.

22. Verfahren zur Produktion erhöhter Mengen an an Verbindungen, die durch den "common pathway" zu einer Zielverbindung fließen, welche ein Intermediat im Weg zwischen DAHP und EPSP ist, durch Züchten, unter geeigneten Nährstoffbedingungen, eines Mikroorganismus, umfassend ein erstes genetisches Element, welches die Menge an funktionellem CsrA-Protein herabsetzt, und ein zweites genetisches Element, welches die Menge an E4P erhöht, wobei der Mikroorganismus zum Kanalisieren von überschüssigem PEP in die Produktion der Zielverbindung unter Bedingungen einer erhöhten Produktion oder nicht-beschränkender Mengen an E4P, DAHP-Synthase und wahlweise anderer Enzyme innerhalb des Wegs, der zu der Zielverbindung führt, angepasst ist.

23. Mikroorganismus, der genetisch angepasst worden ist, um die Synthese von aromatischen Substanzen zu erhöhen, umfassend ein erstes genetisches Element, welches die verfügbare Menge an funktionellem CsrA-Protein herabsetzt, und ein zweites genetisches Element, welches die Menge an D-Erythrose-4-phosphat (E4P) erhöht

## Revendications

1. Micro-organisme comprenant un premier élément génétique qui diminue la quantité disponible de la protéine CsrA fonctionnelle, et un deuxième élément génétique qui augmente la quantité de D-érythrose-4-phosphate (E4P), le micro-organisme étant génétiquement adapté de façon à produire, en présence d'un apport approprié de nutriments, des quantités de phosphoénolpyruvate (PEP) suffisantes pour convertir l'E4P en 3-désoxy-D-arabino-heptulosonate-7-phosphate (DAHP), et le shikimate-3-phosphate obtenu en 5-énolpyruvoylshikimate-3-phosphate (EPSP).

2. Micro-organisme selon la revendication 1, qui est génétiquement adapté de façon à produire une quantité de PEP suffisante pour convertir l'E4P en DAHP et le shikimate-3-phosphate obtenu en EPSP dans des conditions d'une production accrue, ou des quantités non limitées de DAHP-synthase et d'autres enzymes participant à la synthèse de composés qui sont des intermédiaires dans la voie entre le DHAP et l'EPSP.

3. Micro-organisme selon la revendication 1 ou 2, appartenant à l'espèce *E. coli*.

4. Micro-organisme selon l'une quelconque des revendications précédentes, dans lequel le deuxième élément génétique est un plasmide qui code le gène de la transkétolase (tktA).

5. Micro-organisme selon l'une quelconque des revendications précédentes, comprenant un élément génétique additionnel, qui assure une dérépression par rétroaction d'une isozyme de la DAHP-synthase, et/ou qui augmente l'expression de la DAHP-synthase.

6. Micro-organisme selon l'une quelconque des revendications précédentes, qui comprend un premier élément génétique additionnel qui augmente la quantité de shikimate-kinase.

7. Micro-organisme selon la revendication 5 ou 6, dans lequel l'élément génétique additionnel est AroF (FB1), AroG (FB1) ou un mutant *tyrR*.

8. Micro-organisme selon l'une quelconque des revendications précédentes, dans lequel l'apport de nutriments comprend du glucose en tant que source primaire de carbone, ou contient du glucose en une quantité comprise entre 0,2 et 0,4 %.

9. Micro-organisme selon l'une quelconque des revendications précédentes, qui comprend un deuxième élément génétique additionnel qui assure la dérépression de la chorismate-mutase P-préphénate-déhydratase (CMP-PDH).

10. Micro-organisme selon la revendication 6 ou 9, qui assure la dérépression en rétroaction de la CMP-PDH.

11. Micro-organisme selon la revendication 9 ou 10, dans lequel le deuxième élément génétique additionnel est un mutant pheAo ou pheA (FB1).

12. Micro-organisme selon l'une quelconque des revendications précédentes, qui est génétiquement adapté à partir de la souche NST74 appelée ATCC 31884, de la souche mutante de *E. coli* NST 37 et appelée ATCC 31882, ou de la souche mutante de *E. coli* NST 70 et appelée ATCC 31883.

13. Micro-organisme selon la revendication 1, qui est une souche de *E. coli* présentant une production altérée de 3-désoxy-D-arabino-heptulosonate-7-phosphate (DAHP) synthase et de chorismate-mutase P-préphénate-déhydratase (CMP-PDH), dans lequel
i) la DAHP-synthase n'est pas soumise à une inhibition en rétroaction par la phénylalanine, la tyrosine ou le tryptophane présent à l'intérieur du micro-organisme, et la DAHP-synthase
a) n'est pas soumise à une répression par la phénylalanine, la tyrosine ou le tryptophane ; et
b) est produite en une quantité plus grande que celle qui est produite par *E. coli* de type sauvage ; et
ii) la CMP-PDH n'est pas soumise à une inhibition en rétroaction par la phénylalanine, et la CMP-PDH
a) n'est pas soumise à une répression par la phénylalanine ; ou bien
b) est produite en une quantité plus grande que celle qui est produite par *E. coli* de type sauvage.

14. Micro-organisme selon la revendication 1, qui est une souche de *E. coli* ayant une production altérée de shikimate-kinase, dans lequel
i) la shikimate-kinase n'est pas réprimée par la phénylalanine, la tyrosine ou le tryptophane ; ou
ii) la quantité de ladite shikimate-kinase produite est supérieure à celle qui est produite par un *E. coli* de type sauvage.

15. Micro-organisme selon la revendication 1, qui est une souche de *E. coli,* et dans lequel
a) l'ADN du micro-organisme est construit de telle sorte que la DAHP-synthase du micro-organisme soit désensibilisée vis-à-vis d'une inhibition tant par la phénylalanine que par la tyrosine ;
b) la formation de DAHP-synthase dans le micro-organisme est désensibilisée à une répression par mutation du gène régulateur *tyrR* du micro-organisme ; et/ou
c) les voies métaboliques du micro-organisme sont bloquées par mutation du gène tyrA, pour bloquer la production de la tyrosine, ou par une mutation du gène *trpE* pour bloquer la production de tryptophane.

16. Micro-organisme selon la revendication 1, 5 ou 12, qui est une souche de *E. coli* comprenant des plasmides multicopies, chacun des plasmides multicopies ayant un gène de structure pour la production d'au moins un membre du groupe consistant en la DAHP-synthase, la CMP-PDH et la shikimate-kinase.

17. Micro-organisme génétiquement adapté de façon à produire une quantité accrue d'un produit constitué à partir de ressources de carbone s'écoulant par la voie du shikimate en passant par l'EPSP, le micro-organisme comprenant des éléments génétiques choisis au moins parmi les éléments suivants :
a) un premier élément génétique, qui diminue la quantité de la protéine CsrA fonctionnelle ;
b) un deuxième élément génétique qui augmente la quantité de E4P ;
c) un ou plusieurs éléments génétiques additionnels qui assurent la dérépression en rétroaction d'une ou plusieurs isozymes de la DAHP-synthase ;
d) un ou plusieurs éléments génétiques additionnels qui augmentent la quantité de DAHP-synthase ;
e) un ou plusieurs éléments génétiques additionnels qui augmentent la quantité de shikimate-kinase ; et
f) une mutation dans le gène tyrR ;
le micro-organisme comprenant au moins le premier et le deuxième éléments génétiques.

18. Micro-organisme qui est génétiquement adapté de façon à produire des quantités renforcées de composés passant par la voie aromatique usuelle vers un composé cible qui est un intermédiaire de la voie entre le DAHP et l'EPSP, le micro-organisme comprenant un premier élément génétique qui diminue la quantité de la protéine CsrA fonctionnelle, et un deuxième élément génétique qui augmente la quantité de E4P, le micro-organisme étant adapté de façon à canaliser le PEP en excès vers la production du composé cible dans les conditions de quantités accrues ou non limitées de E4P, de DAHP-synthase et de préférence d'autres enzymes dans la voie conduisant au composé cible.

19. Procédé de production de phénylalanine, qui comprend les étapes de culture d'une souche de *E. coli* dans un milieu contenant une source de carbone et de récupération de la phénylalanine, dans lequel la souche de *E. coli* possède :
a) un premier élément génétique qui diminue la protéine CsrA ;
b) un deuxième élément génétique qui augmente le D-érythrose-4-phosphate (E4P) ; et
c) une production altérée de 3-désoxy-D-arabino-heptulosonate-7-phosphate (DAHP) synthase et de chorismate-mutase P-préphénate-déhydratase (CMP-PDH), où
i) la DAHP-synthase n'est pas soumise à une inhibition en rétroaction de la phénylalanine, de la tyrosine ou du tryptophane présent à l'intérieur du micro-organisme, et la DAHP-synthase
a) n'est pas soumise à une répression par la phénylalanine, la tyrosine ou le tryptophane ; ou
b) est produite en une quantité plus grande que celle qui est produite par *E. coli* de type sauvage ; et
ii) la CMP-PDH n'est pas soumise à une inhibition en rétroaction par la phénylalanine, et la CMP-PDH
a) n'est pas soumise à une répression par la phénylalanine ; ou
b) est produite en une quantité supérieure à celle qui est produite par *E. coli* de type sauvage.

20. Procédé selon la revendication 19, dans lequel la source de carbone comprend le glucose, le glycérol, le xylose, le maltose, le lactose, le lactate ou l'acide acétique, ou encore le micro-organisme à ADN a subi une modification plus poussée pour empêcher la production, à partir du chorismate, de produits finals autres que la phénylalanine.

21. Procédé de production d'une quantité accrue de produits métaboliques de la voie du shikimate, comprenant l'EPSP et de préférence le chorismate, par culture, dans des conditions de culture appropriées, en particulier dans des conditions correspondant à des nutriments appropriés, d'un micro-organisme comprenant un élément génétique qui diminue la quantité du produit génique csrA disponible et un produit génétique qui provoque une augmentation de la quantité de E4P, ledit micro-organisme étant génétiquement adapté de façon à produire des quantités de PEP suffisantes pour convertir l'E4P en DAHP et pour convertir le shikimate-3-phosphate ainsi obtenu en EPSP.

22. Procédé de production de quantités accrues de composés passant par la voie usuelle pour arriver à un composé cible qui est un intermédiaire de la voie entre le DAHP et l'EPSP, par culture, dans des conditions appropriées de nutriments, d'un micro-organisme comprenant un premier élément génétique qui diminue la quantité de la protéine CsrA fonctionnelle et un deuxième élément génétique qui augmente la quantité d'E4P, ledit micro-organisme étant génétiquement adapté de façon à canaliser le PEP en excès vers la production dudit composé cible dans les conditions d'une production accrue ou de quantités non limitées de E4P, de DAHP-synthase et en option d'autres enzymes dans la voie conduisant au composé cible.

23. Micro-organisme qui a été génétiquement adapté de façon à renforcer la synthèse de substances aromatiques, comprenant un premier élément génétique qui diminue la quantité disponible de protéine CsrA fonctionnelle et un deuxième élément génétique qui augmente la quantité de D-érythrose-4-phosphate (E4P).
